# EUROPEAN PATENT SPECIFICATION

(11) **EP 3 614 992 B1**
(45) Date of publication and mention of the grant of the patent: **13.10.2021**
(21) Application number: 18714199.9
(22) Date of filing: 22.03.2018
(51) Int. Cl.: A61K 8/27, A61K 8/36, A61K 8/44, A61K 8/49, A61Q 5/00, A61K 8/20

(54) **ANTIDANDRUFF HAIR CARE COMPOSITION COMPRISING PYRITHIONE**
ANTISCHUPPEN-HAARPFLEGEZUSAMMENSETZUNG MIT PYRITHION
COMPOSITION DE SOIN CAPILLAIRE ANTIPELLICULAIRE COMPRENANT DE LA PYRITHIONE

(30) Priority: 26.04.2017 WO PCT/CN2017/082065; 04.07.2017 EP 17179526
(43) Date of publication of application: 04.03.2020
(73) Proprietor: Unilever Global IP Limited, Wirral, CH62 AZD (GB); Unilever IP Holdings B.V., 3013 AL Rotterdam (NL)
(72) Inventor: CHEN, Guoqiang, Shanghai 200335 (CN); HOPTROFF, Michael, John, Bebington Wirral CH63 3JW (GB); JI, Chengdong, Shanghai 200335 (CN); MIAO, Miao, Chengdu 610011 (CN)
(74) Representative: Tansley, Sally Elizabeth
(86) International application number: PCT/EP2018/057298
(87) International publication number: WO 2018/197118

(56) References cited:
- WO-A1-03/088965
- WO-A1-2014/124066
- WO-A1-2014/124068
- US-A- 5 227 156
- US-A1- 2004 213 751
- DATABASE GNPD [Online] MINTEL; 1 February 2015 (2015-02-01), "Anti-Dandruff Smoothing and Brightening Shampoo", XP002775021, Database accession no. 2959539
- DATABASE GNPD [Online] MINTEL; 1 December 2014 (2014-12-01), "Shampoo", XP002775022, Database accession no. 2826423

## Description

### Field of the invention

This invention relates to a hair care composition which provides desired anti-dandruff efficacy with optimum stability of the active materials on hair/scalp to ensure maximum anti-dandruff efficacy. The composition may be delivered as a wash-off composition, e.g. a shampoo or a hair conditioner.

### Background of the invention

Hair care compositions generally provide cleansing or conditioning benefits or a combination of the two. Such compositions typically comprise one or more cleansing surfactants which generally aid in cleaning the hair and/or the scalp free of undesirable soil, particles and fatty matter. Conditioning benefit is achieved by including one or more conditioning agents in the hair care composition. Conditioning benefit is delivered with an oily material being deposited onto the hair resulting in the formation of a film, which makes the hair easier to comb when wet and more manageable when dry.

Additionally, anti-dandruff benefit has been provided through hair care compositions, both through shampoos and through hair conditioners. Dandruff is an issue that affects many people globally. The condition is manifested by the shedding of clumps of dead skin cells from the scalp. These are white in colour and provide an aesthetically displeasing appearance. A factor that is believed to contribute to dandruff is certain members of the Malassezia yeasts. Such a product has to perform as a hair cleansing shampoo or as a hair conditioner, while mitigating the ill-effects of dandruff. Typical anti-dandruff agents used in hair care are metal pyrithione e.g., zinc pyrithione (ZPTO), Octopirox® (piroctone olamine), azole antimicrobials (e.g. climbazole), selenium sulfide and combinations thereof.

Many antidandruff hair care products presently comprise zinc based antidandruff agents like zinc pyrithione (ZPTO, which is a particulate agent). It is found that dissolved intact ZPTO molecules are the sole bioactive form of ZPTO. When hair care product with a zinc based anti-dandruff agent like ZPTO is utilized, there is the so-called ZPTO dissociation and photo-oxidation problem where the stability of the bioactive is compromised with the formation of zinc salts which can lower the antidandruff efficacy. US20040213751 A1 (2004, P&G) discloses a composition comprising: a) an effective amount of pyrithione or a polyvalent metal salt of a pyrithione; b) an effective amount of a zinc-containing layered material which provides an augmentation factor greater than 1. The present invention relates to utilization of ZPTO in the hair care products and to improve the stability of the bioactive. The present inventors, during the course of identifying stabilizers which would protect the dissolved intact ZPTO molecules from photo-oxidation and dissociation, have identified the specific combination of an amino acid and selective zinc compound which could be used to inhibit the photo-oxidation and dissociation of ZPTO, and provide further enhanced stabilization of the ZPTO bioactive. Shampoo comprising ZPTO and zinc compound have been in the market. They generally combine ZPTO with Zn sulphate and amino acid which are usually included at very low concentration. The present inventors have found that when amino acid is included at 1 to 5% by weight synergistic effect could be obtained with zinc compound which is included at 0.1 to 5% by weight for improving the stability of the ZPTO bioactive.

WO14124066 A1 (P&G) discloses hair care composition comprising cationic polymers and anionic particulates for improved deposition of anionic particulates.

WO14124068 A1 (P&G) discloses a method of providing cleanness to hair and/or scalp, for example, providing hair volume and/or providing less oiliness on hair and/or scalp, by applying a hair care composition comprising cationic surfactants, high melting point fatty compounds, metal pyrithione, and metal salts other than metal pyrithiones. The addition of the metal salts in the conditioning composition containing metal pyrithione provide improved cleanness to hair and/or scalp.

WO03088965 A1 (P&G) discloses a method for delivering excess zinc to eukaryotic cells to inhibit the metabolism of the cell, the method comprising treating the cells with a zinc ionophoric material that is capable of delivering a zinc ion across a cellular membrane wherein the zinc ionophoric material is in combination with a zinc containing material and further wherein there is an increase in an intracellular zinc level by 1.5 fold more than would occur in the absence of the zinc ionophoric material.

US5227156 B1 (Amway Corp) discloses antidandruff shampoo containing thiazolinone preservative and pyrithione. The activity of the preservative is maintained by adding a stabilizer comprising a zinc compound.

It is thus an object of the present invention to enhance the stability of the zinc based anti-dandruff bioactive.

### Summary of the invention

According to the first aspect of the present invention there is provided a hair care composition comprising from
(i) 0.01 to 3% by weight zinc pyrithione;
(ii) 1 to 5% by weight amino acid; and
(iii) 0.1 to 5% by weight additional zinc compound.

According to the second aspect of the present invention there is provided a method of maximizing the active stability of a zinc pyrithione for providing enhanced antidandruff benefit to a desired scalp surface comprising the steps of applying a composition of the first aspect on to the desired surface followed by rinsing the surface with water.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. In other words, in specifying any ranges of values, any particular upper value can be associated with any particular lower value.

The disclosure of the invention, as found herein, is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

By 'A Hair Care Composition" as used herein, is meant to include a composition for topical application to hair and/or scalp of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, or bar. Non-limiting examples of such compositions include leave-on hair lotions, creams, and wash-off shampoos, conditioners, shower gels, or toilet bar. The composition of the present invention is preferably a wash-off composition, especially preferred being a shampoo or a conditioner.

The present invention relates to a hair care composition comprising 0.01 to 3% zinc pyrithione; 1 to 5% amino acid; and 0.1 to 5% additional zinc compound.

Zinc pyrithione belongs to the class of insoluble metal pyrithione which may be represented by the following general formula: 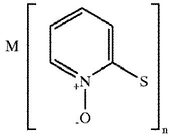 in which M is a polyvalent metal ion and n corresponds to the valency of M. In the present invention M corresponds to Zinc and n has the value of 2.

The zinc pyrithione may have any particle form suitable for use in a composition for topical application. For example, the zinc pyrithione may be in the form of amorphous or crystalline particles having a range of different particle sizes. The zinc pyrithione may, for example, be in the form of particles having a size distribution in which at least about 90% of the particles have a size of up to 100 microns, more preferably up to 50 microns, even more preferably up to 10 microns, most preferably 5 microns or less.

Various methods for producing fine particles of metal pyrithione are described, for example, in EP-A-0 173 259. Suitable methods for determining particle size are described in that document. The insoluble metal pyrithione may be made up of one particulate form or two or more different particulate forms.

Other suitable particulate forms for the zinc pyrithione include platelets and needle-shaped particles. Platelets of zinc pyrithione are described in EP-A-0034385. The needle shaped particles are preferably of the type described in WO99/66886.

For needle-shaped particles preferably at least 50% by number of the particles are needle-shaped particles having a length of between 1 □m and 50 □m. The amount of zinc pyrithione incorporated into the compositions may depend on the type of composition and the exact nature of the material used. Amount of zinc pyrithione is from 0.01 to 3%, preferably from about 0.01 to 1.5% by weight of the total composition, more preferably from 0.05 to 1.5% by weight of the total composition.

The amino acid as per the invention is selected from a wide variety of amino acid which is suitable for use in combination with the essential ingredients of this invention. Suitable amino acids include arginine, histidine, lysine, cysteine, glutamine, glutamic acid, phenylalanine, proline, tryptophan, tyrosine, isoleucine, leucine, methionine, serine and valine.

The amino acids may be added singly, in mixtures, or in the form of peptides, e.g. di- and tripeptides. The amino acids may also be added in the form of a protein hydrolysate, such as a keratin or collagen hydrolysate.

Preferably the amino acid as per the invention is selected from arginine, histidine, glutamine, phenylalanine, proline, serine, tryptophan and tyrosine and mixtures thereof. The most suitable amino acids for the use according to the invention are arginine, histidine and proline.

The amount of amino acid incorporated into the composition may depend on the amount of zinc based anti-dandruff agent used. The amount of amino acid is from 1 to 5% by weight of the total composition.

The composition as per the invention comprises an additional zinc compound. The presence of zinc compound in the composition is believed to have ability to synergistically stabilize ZPTO bioactive in the presence of an amino acid. Thus, as a first essential component there is required a compound, especially a salt, delivering zinc ions. By the term "zinc ion" is meant that the zinc-atom portion of a molecule of the zinc compound in the solid or undissociated state, is capable of being dissociated into simple or complex zinc ions, especially when dispersed in an aqueous medium. Examples of the compounds that may be employed are zinc oxide and zinc salts of the following inorganic ions: borate, bromide, carbonate, hexafluorosilicate, pyrophosphate, silicate, sulphate and titanate. Specific examples include, but are not limited to, zinc oxide, zinc acetate, zinc chloride, zinc citrate, and carbonate.

The amount of zinc compound incorporated into the composition may depend on the amount of zinc based anti-dandruff agent used and type of composition. A preferred amount of zinc compound is from 0.1 to 5%, more preferably from 1 to 5% by weight of the total composition.

As per an especially preferred aspect of the invention, the composition is a shampoo or conditioner.

The composition of the invention especially shampoos are formulated preferably with an anionic surfactant e.g. an alkyl sulphate and/or ethoxylated alkyl sulfate surfactant. These anionic surfactants are preferably present at a level of from 2 to 16%, more preferably from 3 to 16% by weight of the composition. Preferred alkyl sulfates are C8-18 alky sulfates, more preferably C12-18 alkyl sulfates, preferably in the form of a salt with a solubilising cation such as sodium, potassium, ammonium or substituted ammonium. Examples are sodium lauryl sulfate (SLS) or sodium dodecyl sulfate (SDS).

Preferred alkyl ether sulfates are those having the formula: RO (CH2CH2O) nSO¬3M; wherein R is an alkyl or alkenyl having from 8 to 18 (preferably 12 to 18) carbon atoms; n is a number having an average value of greater than at least 0.5, preferably between 1 and 3, more preferably between 2 and 3; and M is a solubilising cation such as sodium, potassium, ammonium or substituted ammonium. An example is sodium lauryl ether sulfate (SLES).

Preferred ethoxylated alkyl sulfate anionic surfactant is sodium lauryl ether sulfate (SLES) having an average degree of ethoxylation of from 0.5 to 3, preferably 1 to 3.

The composition as per the invention optionally and preferably additionally comprises a betaine surfactant. In a preferred embodiment, the composition comprises from 0.1 to 10 wt.%, preferably from 0.5 to 8 wt.%, more preferably from 1 to 5 wt.% of a betaine surfactant, preferably an alkyl amidopropyl betaine, for example cocamidopropyl betaine.

Shampoo compositions according to the invention may comprise one or more further anionic cleansing surfactants which are cosmetically acceptable and suitable for topical application to the hair.

Examples of further suitable anionic cleansing surfactants are the alkaryl sulphonates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in shampoo compositions of the invention include sodium oleyl succinate, ammonium lauryl sulphosuccinate, sodium lauryl ether sulphosuccinate, sodium dodecylbenzene sulphonate, triethanolamine dodecylbenzene sulphonate, lauryl ether carboxylic acid and sodium N-lauryl sarcosinate.

Suitable preferred additional anionic cleansing surfactants are sodium lauryl ether sulphosuccinate(n)EO, (where n is from 1 to 3), lauryl ether carboxylic acid (n) EO (where n is from 10 to 20).

Mixtures of any of the foregoing anionic cleansing surfactants may also be suitable.

If added, the total amount of anionic cleansing surfactant in shampoo compositions of the invention may generally range from 0.5 to 45 wt. %, preferably from 1.5 to 35 wt. %, more preferably from 5 to 20 wt. %, calculated by total weight anionic cleansing surfactant based on the total weight of the composition.

The hair conditioning composition comprises conditioning surfactants selected from cationic surfactants, used singly or in admixture. Preferably, the cationic surfactants have the formula N+R1R2R3R4 wherein R1, R2, R3 and R4 are independently (C1 to C30) alkyl or benzyl. Preferably, one, two or three of R1, R2, R3 and R4 are independently (C4 to C30) alkyl and the other R1, R2, R3 and R4 group or groups are (C1-C6) alkyl or benzyl. More preferably, one or two of R1, R2, R3 and R4 are independently (C6 to C30) alkyl and the other R1, R2, R3 and R4 groups are (C1-C6) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (eg, oleyl). Alkyl groups are optionally ethoxylated on the alkyl chain with one or more ethyleneoxy groups.

Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant. Yet another preferred cationic surfactant is stearamidopropyl dimethylamine.

The most preferred cationic surfactants for use in the composition are stearamidopropyl dimethylamine, behentrimonium chloride, or stearyl trimethyl ammonium chloride. In conditioners of the invention, the level of cationic surfactant will generally range from 0.1 to 5%, preferably 0.5 to 2.5% by weight of the composition.

Hair conditioning compositions of the invention preferably may also additionally comprise a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention will generally range from 0.5 to 10%, preferably from 0.1 to 8%, more preferably from 0.2 to 7 %, most preferably from 0.3 to 6 % by weight of the composition. The weight ratio of cationic surfactant to fatty alcohol is suitably from 1:1 to 1:10, more preferably from 1:1.5 to 1:8, optimally from 1:2 to 1:5.

According to another aspect of the invention there is provided a method of maximizing the stability of zinc pyrithione on to scalp with a uniform stability profile comprising the steps of applying a composition of the invention on to the desired scalp surface followed by rinsing the surface with water. The method is preferably for non-therapeutic benefits.

The invention will now be illustrated with reference to the following Examples.

### Examples

### Example 1-4: Effect of the combination of amino acid and zinc compound as per the invention on the stability of ZPTO bioactive (in solution).

The following compositions having the specific combination of amino aicd and zinc compound as shown in Table-1 were prepared.

**Table-1**

| Components wt% | Zinc pyrithione wt% | Amino acids wt% | Type of Amino acids | Zinc compound wt% | Type of zinc compound | Water and methanol (1:1) wt% |
|---|---|---|---|---|---|---|
| EX A | 0.002 | 0 | - | 0 | | To 100 |
| EX B | 0.002 | 0 | - | 0.01 | Zinc acetate | To 100 |
| EX C | 0.002 | 0 | | 0.01 | Zinc chloride | To 100 |
| EX D | 0.002 | 0.01 | Arginine | 0 | | To 100 |
| EX 1 | 0.002 | 0.01 | Arginine | 0.01 | Zinc acetate | To 100 |
| EX 2 | 0.002 | 0.01 | Arginine | 0.01 | Zinc chloride | To 100 |
| EX E | 0.002 | 0.01 | Histidine | 0 | | To 100 |
| EX 3 | 0.002 | 0.01 | Histidine | 0.01 | Zinc acetate | To 100 |
| EX 4 | 0.002 | 0.01 | Histidine | 0.01 | Zinc chloride | To 100 |

The compositions in Table-1 above were subjected to a procedure that gives an estimate of the residual amount of Zn(PT)₂ active in the hair care composition after being exposed to cool white light for long time. The %average residue of Zn(PT)₂ was measured using an in vitro model as given below.

### Stability test in cool white light model:

The cool white light (CWL) irradiation was carried out in an X-Rite (Macbeth) Spectra Light III chamber. CWL mode was chosen which provides stable irradiation mimicking the office light. The intensity of light was fixed which estimated at 6 µw/cm2 for UVA and 1.5 µw/cm² for UVB. The chamber temperature was equal to the room temperature (20 ± 2 °C). The samples were placed in a line close to the center of the chamber. At different time points (t = 0, 3 and 6 days), the changes of intact ZnPT in samples were quantitatively measured using UPLC-UV analysis following DPS derivatization.

### DPS derivation:

At different time points, 1 mL of solution was taken from each sample for analyzing the amount of intact ZnPT. The solution was firstly diluted with 1 mL of deionized water, and then was mixed with 50 µL of saturated EDTA-2Na and 100 µL of DPS reagent. After 30 minutes of derivatization in dark, the solution was filtered and subjected to UPLC-UV analysis. For the calibration solutions, 1 mL of solution was taken and mixed with 50 µL of saturated EDTA-2Na and 100 µL of DPS reagent. After derivation, the calibration solutions were analyzed in the same way as the sample for the quantification of ZnPT.

### ZnPT analysis by UPLC-UV:

The sample analysis was carried out on a Waters ACQUITY UPLC System coupled to PDA detector. The sample was separated on a Waters ACQUITY C18 column (2.1 mm x 50 mm x 1.7 µm). The mobile phase was composed of methanol (A) and DI water (B) programmed in a gradient. The flow rate was fixed at 0.3 mL/min and total analysis time was 8 min. Column temperature was set at room temperature and the injection volume was 3 µL. The sample was measured by PDA detector at absorption wavelength of 235 nm.

The data of stability of ZPTO in the composition of Table-1 is listed in table-2 below.

**Table-2**

| Stability test of ZPTO | %average⁽¹⁾ residue of Zn(PT)₂ 3 days | SD ⁽²⁾ | % average residue of Zn(PT)₂ 6 days | SD |
|---|---|---|---|---|
| EX A | 60.1 | 0.7 | 31.3 | 0.3 |
| EX B | 71.9 | 0.5 | 46.7 | 0.3 |
| EX C | 72.4 | 0.5 | 47.6 | 0.8 |
| EX D | 62.2 | 0.1 | 33.4 | 0.1 |
| EX 1 | 79.9 | 1.2 | 54.5 | 0.1 |
| EX 2 | 80.2 | 0.1 | 54.6 | 0.6 |
| EX E | 62.0 | 1.0 | 33.3 | 0.4 |
| EX 3 | 76.6 | 0.6 | 52.2 | 0.6 |
| EX 4 | 77.9 | 0.9 | 53.3 | 1.2 |

| | | | | |
|---|---|---|---|---|
| (1) Indicates the average of two readings (2) SD is the standard deviation of the % average residue | | | | |

The data above indicates that compositions as per the invention (Examples 1 to 4) provide for better ZPTO bioactive stability as compared to a corresponding example outside the invention (Examples A to E, not containing either an amino acid or a zinc compound or both) after being exposed to light for long time.

### Example 5 to 10: Effect of combination of different amino acids and zinc compound as per the invention on the stability of ZPTO bioactive (in solution).

The following compositions having the specific combination of amino acids and zinc acetate as shown in Table-3 were prepared.

**Table-3**

| Components wt% | Zinc pyrithione wt% | Amino acids wt% | Type of Amino acids | Zinc acetate wt% | Water and methanol (1:1) wt% |
|---|---|---|---|---|---|
| EX F | 0.002 | 0 | - | 0.01 | To 100 |
| EX 5 | 0.002 | 0.01 | Glutamine | 0.01 | To 100 |
| EX 6 | 0.002 | 0.01 | Phenylalanine | 0.01 | To 100 |
| EX 7 | 0.002 | 0.01 | Proline | 0.01 | To 100 |
| EX 8 | 0.002 | 0.01 | Serine | 0.01 | To 100 |
| EX 9 | 0.002 | 0.01 | Tryptophan | 0.01 | To 100 |
| EX 10 | 0.002 | 0.01 | Tyrosine | 0.01 | To 100 |

The ZPTO residues of the various compositions were measured using an in vitro model as given herein above. The data of stability of ZPTO in the composition of Table-3 is listed in Table-4 below.

**Table 4**

| Stability test of ZPTO | % average residue of Zn(PT)₂ six days | SD |
|---|---|---|
| EX F | 42.0 | 0.2 |
| EX 5 | 43.9 | 0.4 |
| EX 6 | 46.7 | 0.4 |
| EX 7 | 48.0 | 0 |
| EX 8 | 46.2 | 0 |
| EX 9 | 46.5 | 0.3 |
| EX 10 | 46.7 | 0.7 |

The data in Table-4 above indicates that composition as per the invention (Example 5 to 10) provides for better ZPTO stability as compared to an example outside the invention (Examples F).

### Example 11 to 13: Effect of weight percentage of amino acids in the shampoo compositions as per the invention on the stability of ZPTO bioactive (in shampoo formulation).

The following shampoo compositions having the specific combination of amino acids and zinc acetate as shown in Table-5 were prepared.

**Table-5**

| Components wt% | Zinc pyrithione wt% | Tryptophan wt% | Zinc acetate wt% | Carbopol wt% | SLES wt% | CAPB wt% | Water wt% |
|---|---|---|---|---|---|---|---|
| EX G | 1.0 | 0 | 0 | 0.6 | 0.8 | 0.5 | To 100 |
| EX H | 1.0 | 0 | 1.0 | 0.6 | 0.8 | 0.5 | To 100 |
| EX I | 1.0 | 0.1 | 1.0 | 0.6 | 0.8 | 0.5 | To 100 |
| EX J | 1.0 | 0.5 | 1.0 | 0.6 | 0.8 | 0.5 | To 100 |
| EX 11 | 1.0 | 1.0 | 1.0 | 0.6 | 0.8 | 0.5 | To 100 |
| EX 12 | 1.0 | 3.0 | 1.0 | 0.6 | 0.8 | 0.5 | To 100 |
| EX 13 | 1.0 | 5.0 | 1.0 | 0.6 | 0.8 | 0.5 | To 100 |

Dilution of the shampoos prior to stability test: 0.250 g shampoo were added into a 250 mL volumetric flask and the flask was filled with methanol to the mark.

The ZPTO residues of the various compositions were measured using an in vitro model as given herein above. The data of stability of ZPTO in the compositions of Table-5 is listed in Table-6 below.

**Table-6**

| Stability test of ZPTO | % average residue of Zn(PT)₂ six days | SD |
|---|---|---|
| EX G | 28.4 | 0.3 |
| EX H | 32.9 | 0.3 |
| EX I | 31.5 | 0.2 |
| EX J | 34.2 | 0.2 |
| EX 11 | 35.0 | 0.7 |
| EX 12 | 41.1 | 0.3 |
| EX 13 | 46.9 | 0.6 |

The data show that stability of ZPTO increases as the amount of amino acid increases. The results show that the shampoo compositions according to the invention (Example 11 to 13) provide better ZPTO stability.

It is to be understood that some experiments described above were conducted in a test tube at concentration level needed to prove that it is essential to combine zinc pyrithione, amino acid and additional zinc compound to get the desired stability benefit. It is expected that the concentrations to be actually used to prepare a composition for topical use would be vastly different. The concentrations could be orders of magnitude higher due to reasons that affect the difference in concentration in the bulk as compared to that at the cellular level. The composition may be formulated as an emulsion or a gel with very many additional ingredients which affect the concentration of the desired actives - e.g. a water insoluble material like ZPTO, an amino acid or a salt/oxide like a zinc compound, in the oil phase and in the water phase which is expected to be very different. They may also have very different physical and hydrodynamic properties like partition coefficients, diffusional rates, convective transport rates, rheological properties etc. Therefore, it is expected that the concentrations to be used when formulated as a composition would be very different (and typically orders of magnitude higher) from that at the cellular level, at which the experiments were carried out.

## Claims

1. A hair care composition comprising
(i) 0.01 to 3% by weight zinc pyrithione;
(ii) 1 to 5% by weight amino acid; and
(iii) 0.1 to 5% by weight additional zinc compound.

2. A composition as claimed in claim 1 wherein the amino acid is selected from histidine, arginine, glutamine, phenylalanine, proline, serine, tryptophan and tyrosine.

3. A composition as claimed in claim 1 or 2 wherein the zinc compound is selected from zinc oxide, zinc acetate, zinc chloride, zinc citrate or zinc carbonate.

4. A composition as claimed in any one of the preceding claims additionally comprising a surfactant.

5. A composition as claimed in claim 4 wherein the surfactant is an anionic surfactant or a cationic surfactant.

6. A composition as claimed in claim 5 wherein the anionic surfactant is an alkyl sulphate and/or an ethoxylated alkyl sulfate surfactant.

7. A composition as claimed in 6 additionally comprising a betaine surfactant.

8. A composition as claimed in claim 6 or claim 7 wherein the composition is a shampoo.

9. A composition as claimed in claim 5 wherein the cationic surfactant is chosen from stearamidopropyl dimethylamine, behentrimonium chloride, or stearyl trimethyl ammonium chloride.

10. A composition as claimed in claim 9 additionally comprising 0.5 to 10 wt% of a fatty alcohol.

11. A composition as claimed in claim 9 or 10 wherein the composition is a hair conditioner.

12. A method of maximizing the stability of a zinc-based antidandruff agent on to scalp with a uniform stability profile comprising the steps of applying a composition as claimed in any one of the preceding claims on to the desired surface followed by rinsing the surface with water.

## Patentansprüche

1. Haarpflegezusammensetzung, umfassend
(i) 0,01 bis 3 Gewichts-% Zinkpyrithion;
(ii) 1 bis 5 Gewichts-% Aminosäure; und
(iii) 0,1 bis 5 Gewichts-% zusätzliche Zinkverbindung.

2. Zusammensetzung wie in Anspruch 1 beansprucht, wobei die Aminosäure ausgewählt ist aus Histidin, Arginin, Glutamin, Phenylalanin, Prolin, Serin, Tryptophan und Tyrosin.

3. Zusammensetzung wie in Anspruch 1 oder 2 beansprucht, wobei die Zinkverbindung ausgewählt ist aus Zinkoxid, Zinkacetat, Zinkchlorid, Zinkcitrat oder Zinkcarbonat.

4. Zusammensetzung wie in irgendeinem der vorhergehenden Ansprüche beansprucht, die zusätzlich ein Tensid umfasst.

5. Zusammensetzung wie in Anspruch 4 beansprucht, wobei das Tensid ein anionisches Tensid oder ein kationisches Tensid ist.

6. Zusammensetzung wie in Anspruch 5 beansprucht, wobei das anionische Tensid ein Alkylsulfat und/oder ein ethoxyliertes Alkylsulfat-Tensid ist.

7. Zusammensetzung wie in Anspruch 6 beansprucht, zusätzlich umfassend ein Betaintensid.

8. Zusammensetzung wie in Anspruch 6 oder Anspruch 7 beansprucht, wobei die Zusammensetzung ein Shampoo ist.

9. Zusammensetzung wie in Anspruch 5 beansprucht, wobei das kationische Tensid ausgewählt ist aus Stearamidopropyldimethylamin, Behentrimoniumchlorid oder Stearyltrimethylammoniumchlorid.

10. Zusammensetzung wie in Anspruch 9 beansprucht, die zusätzlich 0,5 bis 10 Gew.-% eines Fettalkohols enthält.

11. Zusammensetzung wie in Anspruch 9 oder 10 beansprucht, wobei die Zusammensetzung eine Haarspülung ist.

12. Verfahren zur Maximierung der Stabilität eines Antischuppenmittels auf Zinkbasis auf der Kopfhaut mit einem gleichförmigen Stabilitätsprofil, umfassend die Schritte des Auftragens einer Zusammensetzung wie in irgendeinem der vorhergehenden Ansprüche beansprucht auf die gewünschte Oberfläche, gefolgt vom Spülen der Oberfläche mit Wasser.

## Revendications

1. Composition pour le soin des cheveux comprenant
(i) de 0,01 à 3 % en masse de pyrithione de zinc ;
(ii) de 1 à 5 % en masse d'acide aminé ; et
(iii) de 0,1 à 5 % en masse de composé de zinc supplémentaire.

2. Composition selon la revendication 1, dans laquelle l'acide aminé est choisi parmi l'histidine, l'arginine, la glutamine, la phénylalanine, la proline, la sérine, le tryptophane et la tyrosine.

3. Composition selon la revendication 1 ou 2, dans laquelle le composé de zinc est choisi parmi l'oxyde de zinc, l'acétate de zinc, le chlorure de zinc, le citrate de zinc ou le carbonate de zinc.

4. Composition selon l'une quelconque des revendications précédentes comprenant de plus un tensioactif.

5. Composition selon la revendication 4, dans laquelle le tensioactif est un tensioactif anionique ou un tensioactif cationique.

6. Composition selon la revendication 5, dans laquelle le tensioactif anionique est un sulfate d'alkyle et/ou un tensioactif de sulfate d'alkyle éthoxylé.

7. Composition selon la revendication 6, comprenant de plus un tensioactif de bétaine.

8. Composition selon la revendication 6 ou revendication 7, dans laquelle la composition est un shampooing.

9. Composition selon la revendication 5, dans laquelle le tensioactif cationique est choisi parmi la stéaramidopropyldiméthylamine, le chlorure de béhentrimonium, ou le chlorure de stéaryltriméthylammonium.

10. Composition selon la revendication 9 comprenant de plus de 0,5 à 10 % en masse d'un alcool gras.

11. Composition selon la revendication 9 ou 10, dans laquelle la composition est un après-shampooing.

12. Procédé pour maximiser la stabilité d'un agent antipelliculaire à base de zinc sur un cuir chevelu avec un profil de stabilité uniforme comprenant les étapes d'application d'une composition selon l'une quelconque des revendications précédentes sur la surface souhaitée suivie par le rinçage de la surface avec de l'eau.
